# EUROPÄISCHE PATENTSCHRIFT

(11) **EP 0 374 778 B1**
(45) Veröffentlichungstag und Bekanntmachung des Hinweises auf die Patenterteilung: **06.04.1994**
(21) Anmeldenummer: 89123321.5
(22) Anmeldetag: 16.12.1989
(51) Int. Cl.: C07K 17/00, G01N 33/531, G01N 33/543

(54) **Verfahren zur Proteinimmobilisierung an einer Festphase, so hergestellte Protein tragende Festphase sowie deren Verwendung**
Method for immobilising a protein in a solid phase, the protein-bearing solid phase and its use
Méthode pour l'immobilisation d'une protéine sur une phase solide, la phase solide portant la protéine et son utilisation

(30) Priorität: 19.12.1988 DE 3842700
(43) Veröffentlichungstag der Anmeldung: 27.06.1990
(73) Patentinhaber: BOEHRINGER MANNHEIM GMBH, 68298 Mannheim (DE)
(72) Erfinder: Maier, Josef, D-8120 Weilheim (DE); Mangold, Dieter, Dr. rer. nat., D-6701 Maxdorf (DE); Schlipfenbacher, Reiner, Dr., D-6840 Lampertheim (DE)

(56) Entgegenhaltungen:
- EP-A- 0 122 209
- EP-A- 0 124 320
- EP-A- 0 269 092
- FR-A- 2 280 352

## Beschreibung

Die Erfindung betrifft ein Verfahren zur Proteinimmobilisierung an einer Festphase, eine so hergestellte Protein tragende Festphase sowie deren Verwendung.

An Festphasen immobilisierte Proteine besitzen eine große Bedeutung in verschiedenen Gebieten der Technik. Genannt seien zum Beispiel der Einsatz immobilisierter Proteine in der Biotechnologie, beispielsweise in Bioreaktoren oder in der biochemischen Produktion, z. B. für Affinitätschromatographien.

Eine besondere Bedeutung haben immobilisierte Proteine für die Analytik erlangt. Insbesondere bei Nachweisverfahren von für die medizinische Diagnostik relevanten Parametern in Körperflüssigkeiten, wie Blut, Plasma, Serum, Urin, Speichel, Liquor etc. spielen bioaffine Reaktionen eine wesentliche Rolle, d. h. solche Reaktionen, bei denen eine bestimmte Substanz spezifisch an ein Protein gebunden wird. Bekannte Substanz/Protein-Paare, die solche bioaffinen Reaktionen eingehen, sind in allgemeiner Form z. B. Antigen/Antikorper, Kohlehydrat/Lectin, Biotin/Avidin etc. Ist das Protein an eine Festphase immobilisiert, können in einer Probe vorliegende entsprechende Substanzen, die mit dem Protein spezifisch bindefähig sind, daran gebunden und aus der Probe entfernt und qualitativ und/oder quantitativ bestimmt werden.

Beispielsweise wurde für den Nachweis eines Partners des Antigen/Antikörper-Paares das Prinzip des heterogenen Immunoassays entwickelt. Dem Fachmann sind viele Varianten bekannt. Allen gemeinsam ist, daß ein Partner des Antigen/Antikörper-Paares an eine Festphase gebunden ist und mit diesem immobilisierten Partner eine Abtrennung zumindest eines Teils des entsprechenden Bindungspartners aus der restlichen Probe durchgeführt wird. Die Menge des abgetrennten Bindungspartners bzw. des Teils des nicht abgetrennten, sondern in der Probe verbliebenen Bindungspartners wird dann an der Festphase bzw. in der restlichen Probe ermittelt.

Für die Immobilisierung von Proteinen an Festphasen sind aus dem Stand der Technik eine Reihe von Verfahren bekannt. So kann die Fixierung eines Proteins an eine Festphase durch chemische oder physikalische Kräfte erfolgen. Seit langem sind Methoden zur Herstellung kovalenter Bindungen zwischen einem festen Trägermaterial und daran zu bindenden Proteinen bekannt. Beispielsweise beschreibt EP-A-0 274 911 den Einsatz chemisch reaktiver Kunststoffmembranen, die Proteine direkt kovalent zu binden vermögen. Dieses Verfahren zur Herstellung Protein tragender Festphasen erfordert jedoch eine lange Kontaktzeit zwischen Membran und zu bindendem Protein, um die chemische Reaktion vollständig ablaufen zu lassen. Außerdem müssen nicht mit Protein abgesättigte Aktivstellen in einem zweiten anschließenden Schritt mit einem Inertprotein belegt werden, damit keine freien Membran-Aktivstellen mehr vorhanden sind, die die spätere Verwendung der Proteinbeladenen Festphase negativ beeinflussen könnten.

Bekannte Verfahren, bei denen reaktive Gruppen der Festphase mit einem bifunktionellen Linker gekuppelt werden, an dessen verbleibende freie Funktion dann das zu fixierende Protein kovalent gebunden wird, erfordern noch mehr Zeit und noch mehr Verfahrensschritte. Mit jedem Verfahrensschritt aber steigen auch das Risiko der Produktion von Fehlchargen und die Produktionskosten.

Ein Problem der nicht-kovalenten Fixierung von Protein an eine Festphase ist dessen schwächere Bindung. So können Proteine, die aus einer Lösung an eine Festphase adsorbiert wurden, relativ leicht wieder von dieser abgelöst werden. Vorschläge zur Beseitigung dieses Problems sind bekannt. In DE-A-34 46 636 sind z. B. Verfahren zur Fixierung eines immunreaktiven Stoffes auf einem porösen Trägermaterial beschrieben. Hierbei wird zur Vermeidung des Haftfestigkeitsproblems am Trägermaterial die Fixierung durch Ablaufenlassen einer Immunreaktion zwischen den beiden Partnern einer Immunreaktion, also zwischen Antikörper und Antigen oder Hapten, erreicht. Es wird so ein Immunkomplex-Netz gebildet, das das zu bindende Protein (Antikörper oder Antigen) enthält und an die Festphase bindet. Der Nachteil dieses Fixierungsverfahrens besteht darin, daß außer dem zu bindenden Protein noch ein weiterer teurer Einsatzstoff (Antikörper oder Antigen) benötigt wird. Außerdem muß die Ansatzführung sehr exakt durchgeführt werden, um eine optimale Bindung an die Festphase zu erreichen.

In GB-A-1 505 400 wird vorgeschlagen, ein immunologisch wirksames Protein zu vernetzen und dann an Polystyrol-Latexpartikel zu adsorbieren. Die Adsorption wird in der Latexemulsion durchgeführt. Nach der Bindung eines Teils des Proteins an die Latexpartikel werden diese abzentrifugiert und mehrmals gewaschen. Die Protein tragenden Polystyrol-Partikel werden in gepufferter wässriger Lösung als Suspension aufbewahrt und für Abtrennreaktionen eingesetzt.

EP-A-0 122 209 beschreibt ein Verfahren zur Bindung biologischer Makromoleküle an Festphasen, das darin besteht, die zu fixierenden Makromoleküle zu polymerisieren, mehrere Stunden zusammen mit hydrophoben Trägermaterialien, wie z. B. Polystyrol zu inkubieren und nach der Bindung eines Teils der polymerisierten Makromoleküle an das Trägermaterial dieses vor dem Einsatz bzw. der Lagerung mehrmals zu waschen.

Beide vorgenannten Verfahren führen nicht zu einer zufriedenstellenden Haftung von Proteinen, insbesondere von spezifisch bindefähigen, d. h. bioaffinen Substanzen am Träger. Außerdem sind für die Proteinfixierung an den Träger langwierige und umständliche Inkubations- und Waschschritte notwendig. EP-A-0 269 092 betrifft ein Verfahren, das gegenüber den beiden vorgenannten die Haftung verbessern soll. Hierzu wird das zu fixierende Protein kovalent an ein hydrophobes Trägerprotein gebunden und dieser Komplex an eine hydrophobe Festphase adsorbiert, wobei durch Ausnutzung der hydrophoben Wechselwirkungen zwischen Festphase und Trägerprotein eine besonders vorteilhafte Fixierung erreicht werden soll.

Sämtlichen drei letztgenannten Schriften ist gemeinsam, daß ausschließlich hydrophobe Trägermaterialien zur nicht-kovalenten Fixierung von Proteinen eingesetzt werden. Dies schränkt die Auswahl an Trägermaterialien erheblich ein. Vor allem für die Anwendung Protein tragender Festphasen in wässrigen Flüssigkeiten, wie es biologische Flüssigkeiten alle sind, sind hydrophile Materialien wegen der besseren Benetzbarkeit oft vorzuziehen.

Der vorliegenden Erfindung liegt ausgehend von diesem Stand der Technik die Aufgabe zugrunde, Proteine mittels eines auch im technischen Maßstab einfachen und schnellen Verfahrens fest an in Wasser im wesentlichen unlösliche Trägermaterialien zu binden, um so Protein tragende Festphasen zu erhalten, die breite Verwendungsmöglichkeiten zum Einsatz bei der Bindung und eventuellen Abtrennung spezifisch bindefähiger Substanzen aus flüssigen Proben bieten.

Diese Aufgabe wird durch die in den Ansprüchen gekennzeichnete Erfindung gelöst. So ist das erfindungsgemäße Verfahren zur Immobilisierung eines Proteins an einer Festphase dadurch gekennzeichnet, daß das zu fixierende Protein aggregiert, in einer Flüssigkeit mit einer hydrophilen Festphase kontaktiert und die Festphase nach erfolgtem Kontakt getrocknet wird.

Mittels des erfindungsgemäßen Verfahrens können prinzipiell alle Proteine immobilisiert werden, die chemisch oder physikalisch zu höheren Molekulargewichten aggregiert werden können. Unter chemischer Aggregation ist hierbei jede Molekulargewichtserhöhung zu verstehen, die mittels chemischer Agentien erzielt wird. So können Proteine z. B. durch Zusatz von Carbodiimiden homopolymerisiert werden. Sie können aber beispielsweise auch durch mehrfunktionelle Moleküle, sogenannte Linker, miteinander verknüpft oder vernetzt werden. Die Palette chemischer Linker eröffnet die Möglichkeit hoher Variabilität der Aggregate z. B. hinsichtlich der Zugänglichkeit bestimmter Proteinstellen, sogenannte Epitope, für mit dem Protein spezifisch bindefähige Substanzen oder hinsichtlich der Haftungsfähigkeit des Proteinaggregats an der Festphase. Die Methode, Proteine mittels Linker zu vernetzen, ist dem Fachmann aus dem Stand der Technik bekannt. Beispielsweise beschreiben GB-A-1 505 400, EP-A-0 122 209 und EP-A-0 269 092 solche Verfahren. Für das erfindungsgemäße Verfahren haben sich Linker, wie z. B. Disuccinidyl-suberat, S-Acetyl-mercapto-bernsteinsäureanhydrid oder Maleinimido-hexanoyl-hydroxysuccinimid als vorteilhaft erwiesen. Besonders gut geeignet für die vorliegende Erfindung ist Disuccinidyl-suberat zur Aggregation von Humanalbumin.

Unter physikalischer Aggregation ist jede Molekulargewichtserhöhung zu verstehen, die ohne chemische Agentien erzielt wird. Beispielsweise ist es bekannt, Proteine, wie z. B. Albumin thermisch zu aggregieren und so das Molekulargewicht zu erhöhen (EP-A-0 269 092). Thermisch aggregiertes Albumin ist ein im Sinne der vorliegenden Erfindung besonders gut immobilisierbares Protein.

Wenn die hergestellten Proteinaggregate vor ihrem Aufbringen auf Festphasen z. B. zur Lagerung lyophilisiert werden sollen, empfiehlt es sich gegebenenfalls, wie oft bei der Lyophilisation von Proteinlösungen, diesen vor der Lyophilisation einen Stabilisator zuzusetzen, der die Lagerfähigkeit des lyophilisierten Proteinaggregats und dessen Löslichkeit bei der Rekonstitution mit Wasser oder Puffer erhöht. Art und Menge dieser Substanzen sind von der Art des jeweiligen Proteins abhängig. Generell dürfen diese gegebenenfalls der Proteinaggregat-Lösung zugegebenen Stoffe keinen negativen Einfluß auf das Immobilisierungsverfahren, insbesondere die Bindefähigkeit des Proteinaggregats an die Festphase ausüben. Geeignete Stoffe sind z. B. Saccharose, Trehalose, Mannose, Dextrane und ähnliche Kohlenhydrate, aber auch Proteine, wie Crotein C, Kollagen oder Rinderserum-albumin. Saccharose hat sich als besonders vorteilhaft erwiesen, insbesondere im Falle von mit Disuccinidyl-suberat vernetztem Humanalbumin. Ein typischer Konzentrationsbereich für der zu lyophilisierenden Proteinaggregat-Lösung gegebenenfalls zugesetzte Stabilisatoren und/oder Lösungsvermittler ist 2 bis 20 Gew.-%. Für den vorgenannten Fall der Saccharose-Zugabe zu einer Lösung von mit Disuccinidyl-suberat vernetztem Humanalbumin liegt die vorzugsweise Konzentration zwischen 4 und 10 Gew.-%. Bei Zusatz von Stabilisatoren und/oder Lösungsvermittlern zu der zu lyophilisierenden Proteinaggregat-Lösung ist darauf zu achten, daß bei Verwendung des rekonstituierten Lyophilisats zur Beladung einer Festphase im Sinne der Erfindung die Konzentration an Stabilisator und/oder Lösungsvermittler nicht so hoch sein darf, daß die Bindefähigkeit des Proteinaggregats an die Festphase negativ beeinflußt wird. Beispielsweise sollte im Falle der Immobilisierung von mit Disuccinidyl-suberat vernetztem Humanalbumin die Konzentration an Saccharose in der Proteinaggregat-Lösung weniger als 2 Gew.-% betragen.

Als Proteine sind nach dem erfindungsgemäßen Verfahren nicht nur natürlich vorkommende und aus natürlichen Quellen isolierte Proteine immobilisierbar, sondern auch synthetisch hergestellte. Beispiele für Proteine, die erfindungsgemäß besonders gut immobilisierbar sind, sind Albumin, Immunglobuline, Transferrin und Collagen. Ebenso sind erfindungsgemäß auch solche Proteine an eine Festphase fixierbar, die einen nicht-Proteinteil, wie beispielsweise Biotin oder ein Kohlehydrat gebunden enthalten. Auf diese Art und Weise sind auch Nichtproteine an Festphasen erfindungsgemäß zu immobilisieren, wenn sie zuvor an ein Protein gebunden werden, das für den Nichtproteinteil als Anker an der Festphase wirken kann.

Auf gleiche Weise können natürlich auch Proteine an ein anderes als Anker an der Festphase wirkendes Protein gebunden sein.

Die Festigkeit der erfindungsgemäßen Immobilisierung des Proteins an die Festphase ist abhängig von verschiedenen Faktoren: Einerseits von dem jeweiligen zu fixierenden Protein, andererseits von der Art des Trägermaterials. Bezüglich des Proteins hat es sich gezeigt, daß die Haftung an der Festphase mit zunehmendem Molekulargewicht zunimmt. Es läßt sich keine generelle Mindestgröße zur Erzielung einer bestimmten Bindefestigkeit angeben. Sie hängt von der Art des jeweils zu bindenden Proteins ab und ist für den Fachmann leicht ermittelbar. Für Albumin hat sich beispielsweise ein Aggregat aus 4 bis 5 Albumineinheiten bereits als gut immobilisierbar im Sinne der Erfindung erwiesen, ein Aggregat aus mindestens 10 Albumineinheiten ist besonders vorteilhaft.

Bevorzugte Festphasen im Sinne der Erfindung sind alle diejenigen in Wasser im wesentlichen unlöslichen Materialien, die hydrophiler sind als das zu immobilisierende aggregierte Protein. Geeignete Materialien sind z. B. Polyester, Sulphitzellstoff, Zellwolle, Linters, Nitrocellulose, Celluloseacetat oder Festphasen auf Nylonbasis. Sie können in beliebiger Form vorliegen, wie beispielsweise Pulver, Körnchen, Fasern, Vliesen oder Folien. Besonders bevorzugt als Festphasen im Sinne der Erfindung sind Vliese auf Cellulose-Basis.

Gemäß dem Verfahren der vorliegenden Erfindung wird das aggregierte Protein in einer Flüssigkeit mit der Festphase kontaktiert. Das aggregierte Protein liegt dabei in gelöster Form vor, vorzugsweise in einer wässrigen Flüssigkeit, beispielsweise einer Pufferlösung. Werden Vliese als Festphasen eingesetzt, hat es sich als besonders vorteilhaft erwiesen, diese mit einer Lösung des aggregierten Proteins zu tränken, indem sie in die Lösung getaucht und darin mit der Lösung gesättigt werden.

Als Pufferlösungen können dabei all diejenigen eingesetzt werden, die dem Fachmann als das jeweilige Protein und dessen biologische Aktivität nicht schädigend bekannt sind. Beispielsweise haben sich für Humanalbumin Phosphat- oder HEPES-Puffer mit einem pH-Wert zwischen ca. 6 und ca. 9, vorzugsweise zwischen ca. 7 und ca. 8 als besonders geeignet als Lösungsmittel erwiesen.

Zur Erzielung einer guten Haftung des aggregierten Proteins an der Festphase ist es wichtig, daß nach der Kontaktierung des Proteinaggregats mit der Festphase die das Protein tragende Festphase getrocknet wird. Unter Trocknung werden hierbei alle speziellen Bemühungen verstanden, Flüssigkeit weitest möglich zu entfernen. Als trocken im Sinne der Erfindung wird im allgemeinen eine hydrophile Festphase mit einem Restfeuchtegehalt von weniger als 7 so bei 20 °C und 50 % relativer Feuchte bezeichnet. Der Restfeuchtegehalt, ab dem von "trocken" geredet werden kann, ist im speziellen Fall abhängig von der ausgewählten Festphase. So sind z. B. Cellulose-Vliese bei Restfeuchtegehalten von weniger als 7 %, Mischvliese aus Cellulose und Polyesterfasern im Gewichtsverhältnis 50:50 von weniger als 5 % als trocken anzusprechen. Um solche Trocknungsgrade bei hydrophilen Festphasen in wirtschaftlich vertretbaren Trocknungszeiten zu erreichen, muß in der Regel Wärme zugeführt werden. So wird nach dem erfindungsgemäßen Verfahren die Trocknung Protein-tragender Vliese vorteilhafterweise bei Temperaturen zwischen 30 und 80 °C durchgeführt. Sind die Trocknungszeiten nicht entscheidend für die Wahl des Verfahrens kann die Trocknung auch bei Raumtemperatur z. B. im Luftstrom durchgeführt werden.

Es wurde überraschenderweise festgestellt, daß die Erreichung eines so geringen Restfeuchtegehaltes der das Protein tragenden Festphase entscheidend für die ausgezeichnete Haftung des Proteins am Träger ist. Höhere Restfeuchtegehalte führen zu einer schwächeren Fixierung des Proteins an der Festphase.

Der besondere Vorteil des erfindungsgemäßen Verfahrens liegt in seiner Einfachheit. Es reicht aus, das hydrophile Trägermaterial, insbesondere hydrophile Vliese, in eine Lösung des zu fixierenden aggregierten Proteins zu tauchen, um es mit Flüssigkeit zu sättigen. Nach erfolgter Tränkung wird das Trägermaterial aus der Proteinlösung genommen und getrocknet. Nach dem Trocknen ist das Proteinaggregat fest an die Festphase gebunden. Auf diese Art und Weise ist es möglich, eine homogene Beladungsdichte der Festphase mit Protein zu erreichen, wobei die Menge an immobilisiertem Protein genau bekannt ist.

Die Menge an Protein, die so an eine Festphase gebunden werden kann, ist wesentlich höher als es durch Adsorption erklärbar ist. So kann dann, wenn nach Durchführung des erfindungsgemäßen Verfahrens nach der Trocknung durch einen zusätzlichen Waschschritt kein Protein von der Festphase eluierbar ist, ohne Trocknung, d. h. wenn eine einfache Adsorption des Proteinaggregats an die Festphase ermöglicht wurde, der Großteil des adsorbierten Proteins durch Waschung wieder eluiert werden. Nach dem erfindungsgemäßen Verfahren erübrigt sich deshalb ein Waschschritt, wenn die Konzentration der Proteinlösung so gewählt wird, daß das gesamte Protein nach der Trocknung immobilisiert ist. Diese Proteinkonzentration ist von der Art des jeweiligen Trägermaterials abhängig. Die Grenzkonzentration, die gerade noch vollständig immobilisiert wird, kann durch einfache Versuche leicht ermittelt werden.

Ein weiterer Vorteil des erfindungsgemäßen Verfahrens liegt darin, daß die Zusammensetzung der Tränklösung in weiten Bereichen frei wählbar ist, ohne die Bindungseigenschaften des zu immobilisierenden Proteins nachteilig zu beeinflussen. So können z. B. Art und Konzentration des Puffers, pH, Ionenstärke und gegebenenfalls zuzusetzende Stabilisatoren und/oder Lösungsvermittler überwiegend nach Gesichtspunkten gewählt werden, die allein die Eigenschaften des jeweiligen gelösten Proteinaggregats betreffen. Auf die Festigkeit der Bindung des immobilisierten Proteinaggregats hat diese Wahl meist keinen Einfluß.

Nach dem erfindungsgemäßen Verfahren hergestellte Festphasen eignen sich sehr gut für die Bindung von mit dem immobilisierten Protein spezifisch bindefähigen Substanzen und gegebenenfalls deren Entfernung aus Flüssigkeiten. Es sind die unterschiedlichsten Anwendungsbereiche denkbar, wie z. B. für die biochemische Produktion als Träger immobilisierter Proteine in Bioreaktoren oder für die Affinitätschromatographie zur Anreicherung und/oder Abtrennung von spezifisch bindefähigen Substanzen.

Besonders bevorzugt werden erfindungsgemäß hergestellte Proteintragende Festphasen für analytische Bestimmungen von Inhaltsstoffen in flüssigen Proben eingesetzt. Ganz besonders bevorzugt werden sie für bioaffine Bestimmungen von Inhaltsstoffen in Körperflüssigkeiten, wie z. B. Blut, Plasma, Serum, Urin, Liquor, Speichel etc., wie sie einleitend beschrieben wurden. Für heterogene Immunoassays eignen sich die erfindungsgemäßen Proteintragenden Festphasen besonders gut, insbesondere für solche, die insgesamt trägergebunden, z. B. auf einem Teststreifen, durchgeführt werden.

Im folgenden wird die Erfindung anhand einiger Beispiele näher erläutert.

### Beispiel 1

### Vernetzung von Humanserum-Albumin (HSA) mittels Disuccinidylsuberat (DSS) zu poly-Humanserum-albumin (pHSA)

1,5 g HSA werden in 30 ml Kaliumphosphat-Puffer, 200 mM, pH = 8,0 vorgelegt und innerhalb 2 Stunden mit 2,5 ml einer Lösung aus 50 mg DSS/ml Dioxan versetzt. Nach Ablauf der Vernetzungsreaktion wird gegen das 500fache Volumen Kaliumphosphat-Puffer, 20 mM, pH 7,2 dialysiert. Die hochmolekulare Fraktion (pHSA) mit einem Molekulargewicht von mehr als 650 000 Dalton wird an Superose 6^{R} (Pharmacia, Freiburg, Bundesrepublik Deutschland) über Gelfiltration abgetrennt und nach Zugabe von 6 mg Saccharose/mg Protein lyophilisiert.

### Beispiel 2

### Erfindungsgemäße Immobilisierung von Humanserum-Albumin und Vergleich der Haftfestigkeit des so immobilisierten Proteins mit adsorbiertem Protein

6 x 6 mm² große und 0,5 mm dicke Vliesstücke aus 50 % Polyester/50 % Linters werden mit 15 µl einer Lösung von 250 mg/l pHSA aus Beispiel 1 in 10 mM Natriumphosphat-Puffer pH 7,5 getränkt und
a) 30 Minuten bei 50 °C getrocknet und dreimal mit 25 µl Natriumphosphat-Puffer (10 mM, pH 7,5) jeweils in der Zentrifuge gewaschen und anschließend ausgeschleudert bzw.
b) nach 10 Minuten in einer Zentrifuge ausgeschleudert und zweimal mit je 25 µl Natriumphosphat-Puffer (10 mM, pH 7,5) gewaschen.

Die Waschzentrifugate für a) und b) werden jeweils vereint und jeweils mit 300 mU anti-HSA-IgG-β-Galactosidasekonjugat (160 µl) versetzt und 10 Minuten geschüttelt. Dann werden je 200 µl in Mikrotiterplatten-Löcher transferiert, deren Wand mit HSA beladen ist. Nach einstündiger Inkubation wird mit Phosphat-gepufferter physiologischer Kochsalzlösung gewaschen, mit o-Nitrophenylgalactosid entwickelt und nach 10 Minuten die Extinktion bei 405 nm vermessen. Die quantitative Bestimmung des aus dem Vlies eluierten pHSA erfolgt über eine mitgeführte Eichkurve.

Die pHSA-Menge mit der jedes Vliesstück kontaktiert wurde, betrug 3,75 µg. Nach der Elution wurden folgende Mengen in den vereinten Waschzentrifugaten ermittelt:

| Verfahren | pHSA im Eluat | Eluat bezogen auf Tränklösung |
|---|---|---|
| a) | 0,012 µg | 0,3 % |
| b) | 2,64 µg | 71 % |

Das erfindungsgemäß immobilisierte Protein ist aufgrund des Trocknungsschrittes wesentlich fester an die Festphase gebunden als das lediglich adsorbierte Protein, das zum größten Teil wieder von der Festphase abgewaschen werden kann.

### Beispiel 3

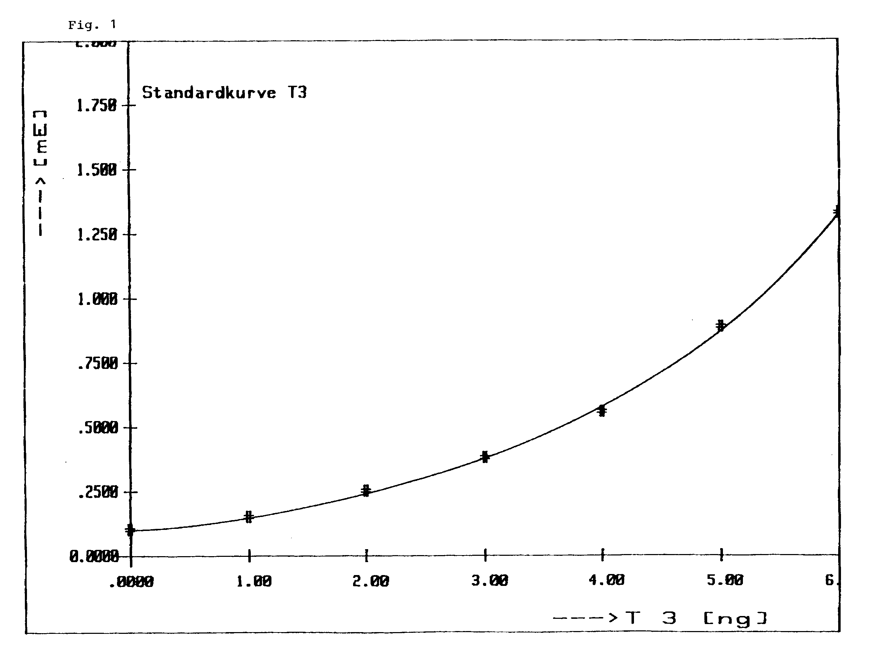

**a) Herstellung und Vernetzung von Kaninchen-IgG-T3-Konjugat**
   3 g Kaninchen-IgG werden in 300 ml Kaliumphosphat-Puffer, 100 mM, pH 8,5 gelöst und mit einer Lösung aus 58 mg N-tert.-butyloxycarbonyl-trijodthyronin-N'-hydroxysuccinimid-ester (BOC-T3-N'-hydroxysuccinimid-ester) in 30 ml Dioxan versetzt.
   Nach zwei Stunden Reaktionszeit wird die Proteinlösung gegen das 200fache Volumen 20 mM Kaliumphosphat-Puffer, pH 7,8 dialysiert und über Ultrafiltration (Amicon YM 100^{R}-Membran) auf eine Konzentration von 50 mg/ml eingestellt.
   Zur Vernetzung werden dem Ansatz langsam 35 ml Disuccinidyl-Suberat (Konzentration 10 mg Disuccinidyl-Suberat/ml Dioxan) zugegeben. Nach Beendigung der Vernetzungsreaktion wird gegen das 500fache Volumen Kaliumphosphat-Puffer, 50 mM, pH 7,2 dialysiert und die an Superose 6^{R} im Ausschlußvolumen eluierte Fraktion abgetrennt sowie mit Saccharose (Konzentration 6 mg Saccharose/mg IgG) stabilisiert und lyophilisiert.
**b) Trijodthyronin (T3)-Test**
   Ein Vlies aus 60 % Sulfit-Zellstoff und 40 % Linters wird mit vernetztem Kaninchen-IgG-T3-Konjugat aus Beispiel 3 a) in einer Konzentration von 250 µg/ml in 50 mM Kaliumphosphat-Puffer, pH 7,2 getränkt und nach vollständiger Sättigung bei 50 °C 60 Minuten lang in einem Umluftschrank getrocknet. Jeweils 50 µl Schaf-anti-T3-IgG-β-Galactosidase-Konjugat (hergestellt analog in J. Immunoassay 4 (1983) 209 - 327 beschriebenen Verfahren) (120 mU in Phosphatgepufferter physiologischer Kochsalzlösung, 5 g/l Rinderserum-Albumin) werden 5 Minuten lang mit 50 µl einer T3-Standardreihe inkubiert und anschließend auf vorstehend hergestellte Vliesstücke (8 x 8 mm, Dicke 0,5 mm) der T3-Matrix pipettiert. Nach 5 Minuten Inkubationszeit werden die Vliesstücke auszentrifugiert und die β-Galactosidaseaktivität des Filtrates mit 40 mM Chlorphenolrotgalactosid-Lösung (hergestellt nach EP-A 0 146 866) bei 578 nm bestimmt.
   Es wird die in Figur 1 dargestellte Standardkurve erhalten, die für die Prüfung von Lösungen mit unbekanntem T3-Gehalt herangezogen werden kann.

### Beispiel 4

### Herstellung und Vernetzung von Kaninchen-IgG-Diaoxigenin-Konjugat

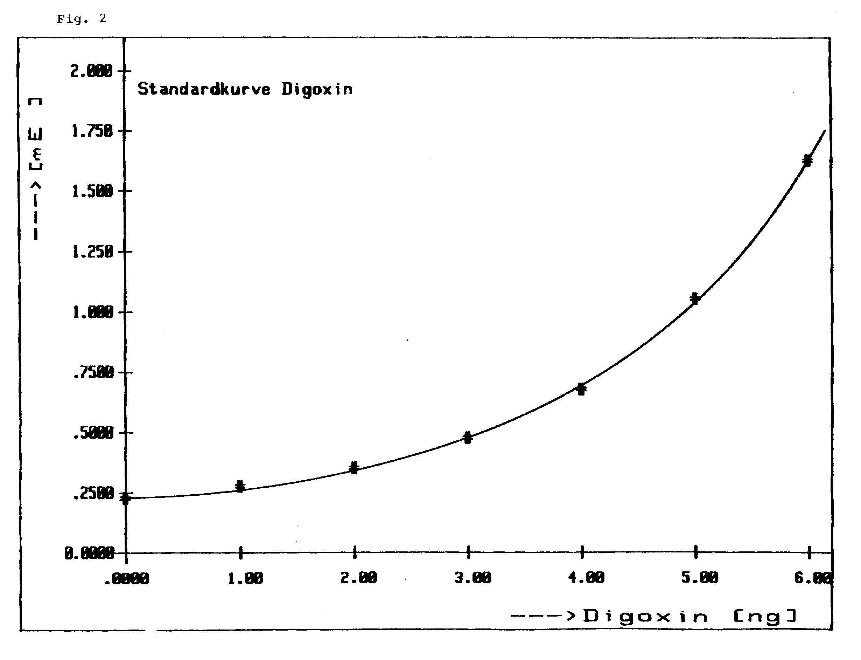

a) 3 g Kaninchen-IgG werden in 300 ml Kaliumphosphat-Puffer, 100 mM, pH 8,5, gelöst und mit einer Lösung aus 58 mg Digoxigenin(3-succinidyl)-N-hydroxysuccinimid in 30 ml Dioxan versetzt. Nach 2 Stunden Reaktionszeit wird die Proteinlösung gegen das 200fache Volumen 20 mM Kaliumphosphat-Puffer pH 7,0 dialysiert und über Ultrafiltration (Amicon YM 100^{R}-Membran) auf eine Konzentration von 50 mg/ml eingestellt.
   Zur Vernetzung werden dem Ansatz langsam 35 ml Disuccinidyl-Suberat-Lösung (Konzentration 10 mg Disuccinidyl-Suberat/ml Dioxan) zugegeben. Nach Beendigung der Vernetzungsreaktion wird gegen das 500fache Volumen Kaliumphosphat-Puffer 50 mM, pH 7,2 dialysiert, die an Superose 6^{R} im Ausschlußvolumen eluierte Fraktion abgetrennt, mit Saccharose (Konzentration 6 mg Saccharose/mg IgG) stabilisiert und lyophilisiert.
**b) Digoxin-Test**
   Ein Vlies aus 60 % Sulfit-Zellstoff und 40 % Linters wird mit vernetztem Kaninchen-IgG-Digoxigenin-Konjugat aus Beispiel 4 a) (250 µg/ml in 50 mM Kaliumphosphat-Puffer, pH 7,2) bis zur Sättigung getränkt und bei 50 °C 60 Minuten lang in einem Umluftschrank getrocknet.
   Jeweils 50 µl Schaf-anti-Digoxin-IgG-β-Galactosidase-Konjugat (hergestellt analog in J. Immunoassay 4 (1983) 209 - 327 beschriebenen Verfahren) (120 mU in Phosphat-gepufferter physiologischer Kochsalzlösung, 5 g/l Rinderserum-Albumin) werden 5 Minuten lang mit 50 µl einer Digoxin-Standardreihe inkubiert und anschließend auf die vorstehend hergestellten Vliesstücke (8 x 8 mm, Dicke 0,5 mm) der Digoxin-Matrix pipettiert. Nach 5 Minuten Inkubationszeit werden die Vliesstücke jeweils in einer Zentrifuge ausgeschleudert und die β-Galactosidaseaktivität des Zentrifugates mit 40 mM Chlorphenolrotgalactosid-Lösung (hergestellt nach EP-A 0 146 866) bei 578 nm bestimmt.
   Es wird die Standardkurve der Figur 2 erhalten, mittels der der unbekannte Digoxingehalt in einer Lösung bestimmt werden kann.

### Beispiel 5

### Imprägnierung von Papier mit thermisch vernetztem Rinderserumalbumin-Streptavidin-Konjugat (tBSA-SA) und Bestimmung der Desorptionsrate

1. Imprägnierung
   8 mm x 8 mm große Quadrate aus Papier (80 % Polyester / 20 % Cellulose / 20 % (bezogen auf Fasern) Etadurin) werden mit 42 µl einer Lösung von 0,5 µg/µl tBSA-SA (Boehringer Mannheim GmbH, Mannheim, Bundesrepublik Deutschland) in 50 mMol/l Kaliumphosphatpuffer, pH 7,0 imprägniert. Anschließend wird 30 Minuten bei 70 °C getrocknet.
2. Bestimmung der Desorptionsrate
   Ein unter 1. hergestelltes Vlies wird 15 Minuten lang in 1 ml 50 mM Kaliumphosphatpuffer, pH 7,0 bewegt. Der Überstand wird in ein 1 ml Enzymun-Plastikröhrchen (Boehringer Mannheim GmbH, Mannheim, Bundesrepublik Deutschland), das innen mit 1 µg/ml tBSA-Biotin 1:1 (Boehringer Mannheim GmbH, Mannheim, Bundesrepublik Deutschland) beschichtet ist, überführt und eine Stunde lang inkubiert. Nach zweimaligem Waschen mit Wasser wird 1 ml einer Peroxidase-Biotin-Konjugatlösung (200 mU/ml, Boehringer Mannheim GmbH, Mannheim, Bundesrepublik Deutschland) in das Röhrchen gegeben und 30 Minuten inkubiert. Nach zweimaligem Waschen mit Wasser wird 2,2'-Azino-di-[3-ethylbenzthiazolinsulfonat(6)] in das Röhrchen gegeben und die Reaktionslösung bei 405 nm vermessen. Das System wird mit einer Standardreihe von Lösungen bekannter tBSA-SA-Konzentration kalibriert.
   Bei Verwendung des nach 1. hergestellten imprägnierten Papiers werden 18 ng tBSA-SA, das entspricht 0,09 % desorbiert. Wird bei dem Imprägnierverfahren nach 1. der Trockenschritt weggelassen, werden 560 ng tBSA-SA, das sind 2,7 % desorbiert. Hieraus folgt, daß das erfindungsgemäß immobilisierte Protein aufgrund des Trocknungsschrittes wesentlich fester an die Festphase gebunden ist als das lediglich adsorbierte Protein.

## Patentansprüche

1. Verfahren zur Immobilisierung eines Proteins an einer Festphase, dadurch gekennzeichnet, daß das zu immobilisierende Protein aggregiert, in einer Flüssigkeit mit einer hydrophilen Festphase kontaktiert und die Festphase nach erfolgtem Kontakt getrocknet wird.

2. Verfahren gemäß Anspruch 1, dadurch gekennzeichnet, daß das Protein durch Homopolymerisierung aggregiert wird.

3. Verfahren gemäß Anspruch 1, dadurch gekennzeichnet, daß das Protein mittels mehrfach-funktioneller Linker aggregiert wird.

4. Verfahren gemäß einem der Ansprüche 1 - 3, dadurch gekennzeichnet, daß zur Immobilisierung eine Trocknung der das Protein enthaltenden Festphase bis zu einem Restfeuchtegehalt von weniger als 7 % bei 20 °C und 50 % relativer Luftfeuchte erfolgt.

5. Verfahren gemäß einem der Ansprüche 1 - 4, dadurch gekennzeichnet, daß nach der Kontaktierung der Festphase mit dem aggregierten Protein kein Waschschritt erfolgt.

6. Verwendung einer nach einem der Ansprüche 1 - 5 hergestellten Protein gebunden enthaltenden Festphase für analytische Bestimmungen.

7. Protein nicht kovalent gebunden enthaltende Festphase, die nach einem der Ansprüche 1 - 5 hergestellt wurde.

## Claims

1. Process for the immobilisation of a protein on a solid phase, characterised in that the protein to be immobilised is aggregated, contacted in a liquid with a hydrophilic solid phase and the solid phase, after contact has taken place, is dried.

2. Process according to claim 1, characterised in that the protein is aggregated by homopolymerisation.

3. Process according to claim 1, characterised in that the protein is aggregated by means of multi-functional linkers.

4. Process according to one of claims 1 - 3, characterised in that, for the immobilisation, a drying of the solid phase containing the protein to a residual moisture content of less than 7% takes place at 20°C and 50% relative atmospheric humidity.

5. Process according to one of claims 1 - 4, characterised in that, after the contacting of the solid phase with the aggregated protein, no washing step takes place.

6. Use of a solid phase containing bound protein prepared according to one of claims 1 - 5 for analytical determinations.

7. Solid phase containing non-covalently bound protein which has been prepared according to one of claims 1 to 5.

## Revendications

1. Procédé permettant d'immobiliser une protéine sur une phase solide, caractérisé par la formation d'agrégats de la protéine à fixer, la mise en contact de ceux-ci avec une phase solide hydrophile dans un liquide et le séchage de la phase solide lorsque le contact a été obtenu.

2. Procédé selon la revendication 1, caractérisé en ce que la formation d'agrégats de la protéine est produite par homopolymérisation.

3. Procédé selon la revendication 1, caractérisé en ce que la formation d'agrégats de la protéine est obtenue à l'aide d'un linker multifonctionnel.

4. Procédé selon l'une des revendications 1-3, caractérisé en ce que, pour réaliser l'immobilisation, on sèche la phase solide contenant la protéine jusqu'à ce que la teneur en eau résiduelle soit inférieure à 7 % à 20°C, en présence d'une humidité de l'air relative de 50 %.

5. Procédé selon l'une des revendications 1-4, caractérisé en ce que, après la mise en contact de la phase solide avec la protéine sous forme d'agrégats, il n'y a pas d'étape de lavage.

6. Utilisation d'une phase solide contenant une protéine fixée, obtenue selon l'une des revendications 1-5, pour des déterminations analytiques.

7. Phase solide contenant une protéine non fixée par liaison covalente, qui a été obtenue selon l'une des revendications 1-5.
